# EUROPÄISCHE PATENTSCHRIFT

(11) **EP 2 306 203 B1**
(45) Veröffentlichungstag und Bekanntmachung des Hinweises auf die Patenterteilung: **11.07.2012**
(21) Anmeldenummer: 10191423.2
(22) Anmeldetag: 08.05.2008
(51) Int. Cl.: G01N 33/74

(54) **Diagnose und Risikostratifizierung mittels NT-proET-1**
Diagnosis and risk stratification using NT-proET-1
Diagnostic et stratification du risque au moyen de NT-proET-1

(30) Priorität: 08.05.2007 DE 102007021443
(43) Veröffentlichungstag der Anmeldung: 06.04.2011
(62) Teilanmeldung aus: 08758039.5
(73) Patentinhaber: B.R.A.H.M.S GmbH, 16761 Hennigsdorf (DE)
(72) Erfinder: Bergmann, Andreas, 12351, Berlin (DE); Struck, Joachim, 13465, Berlin (DE)
(74) Vertreter: Simandi, Claus

(56) Entgegenhaltungen:
- EP-A- 1 564 558
- STRUCK ET AL: "Proteolytic processing pattern of the endothelin-1 precursor in vivo", PEPTIDES, ELSEVIER, AMSTERDAM, Bd. 26, Nr. 12, 1. Dezember 2005 (2005-12-01), Seiten 2482-2486, XP005174851, ISSN: 0196-9781
- PAPASSOTIRIOU JANA ET AL: "Immunoluminometric assay for measurement of the C-terminal endothelin-1 precursor fragment in human plasma", CLINICAL CHEMISTRY, Bd. 52, Nr. 6, Juni 2006 (2006-06), Seiten 1144-1151, XP002484207, ISSN: 0009-9147
- GIAID ADEL ET AL: "Expression of endothelin-1 in the lungs of patients with pulmonary hypertension", NEW ENGLAND JOURNAL OF MEDICINE, Bd. 328, Nr. 24, 1993, Seiten 1732-1739, XP008098918, ISSN: 0028-4793
- STEWART D J ET AL: "INCREASED PLASMA ENDOTHELIN-1 IN PULMONARY HYPERTENSION MARKER OR MEDIATOR OF DISEASE", ANNALS OF INTERNAL MEDICINE, Bd. 114, Nr. 6, 1991, Seiten 464-469, XP008098981, ISSN: 0003-4819
- HUMBERT MARC ET AL: "Cellular and molecular pathobiology of pulmonary arterial hypertension", JOURNAL OF THE AMERICAN COLLEGE OF CARDIOLOGY, Bd. 43, Nr. 12, Suppl. S, 16. Juni 2004 (2004-06-16), Seiten 13S-24S, XP008098866, ISSN: 0735-1097

### SEQUENCE LISTING

<110> BRAHMS GmbH
<120> Diagnose und Risikostratifizierung mittels NT-proET-1
<130> 03/07 BRA35WOEP-02
<140> PCT/DE2008/000781
   <141> 2008-05-08
<150> 10 2007 041 443.1
   <151> 2007-05-08
<160> 6
<170> PatentIn version 3.3
<210> 1
   <211> 212
   <212> PRT
   <213> Human
<400> 1
   Met Asp Tyr Leu Leu Met Ile Phe Ser Leu Leu Phe Val Ala Cys Gln
   1               5                   10                  15
   Gly Ala Pro Glu Thr Ala Val Leu Gly Ala Glu Leu Ser Ala Val Gly
               20                  25                  30
   Glu Asn Gly Gly Glu Lys Pro Thr Pro Ser Pro Pro Trp Arg Leu Arg
           35                  40                  45
   Arg Ser Lys Arg Cys Ser Cys Ser Ser Leu Met Asp Lys Glu Cys Val
       50                  55                  60
   Tyr Phe Cys His Leu Asp Ile Ile Trp Val Asn Thr Pro Glu His Val
   65                  70                  75                  80
   Val Pro Tyr Gly Leu Gly Ser Pro Arg Ser Lys Arg Ala Leu Glu Asn
                   85                  90                  95
   Leu Leu Pro Thr Lys Ala Thr Asp Arg Glu Asn Arg Cys Gln Cys Ala
               100                 105                 110
   Ser Gln Lys Asp Lys Lys Cys Trp Asn Phe Cys Gln Ala Gly Lys Glu
           115                 120                 125
   Leu Arg Ala Glu Asp Ile Met Glu Lys Asp Trp Asn Asn His Lys Lys
       130                 135                 140
   Gly Lys Asp Cys Ser Lys Leu Gly Lys Lys Cys Ile Tyr Gln Gln Leu
   145                 150                 155                 160
   Val Arg Gly Arg Lys Ile Arg Arg Ser Ser Glu Glu His Leu Arg Gln
                   165                 170                 175
   Thr Arg Ser Glu Thr Met Arg Asn Ser Val Lys Ser Ser Phe His Asp
               180                 185                 190
   Pro Lys Leu Lys Gly Lys Pro Ser Arg Glu Arg Tyr Val Thr His Asn
           195                 200                 205
   Arg Ala His Trp
       210
<210> 2
   <211> 14
   <212> PRT
   <213> Human
<400> 2
   Ala Pro Glu Thr Ala Val Leu Gly Ala Glu Leu Ser Ala Val
   1 5 10
<210> 3
   <211> 21
   <212> PRT
   <213> Human
<400> 3
   Gly Glu Asn Gly Gly Glu Lys Pro Thr Pro Ser Pro Pro Trp Arg Leu
   1 5 10 15
   Arg Arg Ser Lys Arg
   20
<210> 4
   <211> 15
   <212> PRT
   <213> Artificial
<220>
   <223> synthetic derivat
<400> 4
   Cys Ala Pro Glu Thr Ala Val Leu Gly Ala Glu Leu Ser Ala Val
   1 5 10 15
<210> 5
   <211> 22
   <212> PRT
   <213> artificial
<220>
   <223> synthetic derivat
<400> 5
   Gly Glu Asn Gly Gly Glu Lys Pro Thr Pro Ser Pro Pro Trp Arg Leu
   1 5 10 15
   Arg Arg Ser Lys Arg Cys
   20
<210> 6
   <211> 35
   <212> PRT
   <213> Human
<400> 6
   Ala Pro Glu Thr Ala Val Leu Gly Ala Glu Leu Ser Ala Val Gly Glu
   1               5                   10                  15
   Asn Gly Gly Glu Lys Pro Thr Pro Ser Pro Pro Trp Arg Leu Arg Arg
               20                  25                  30
   Ser Lys Arg
           35

## Beschreibung

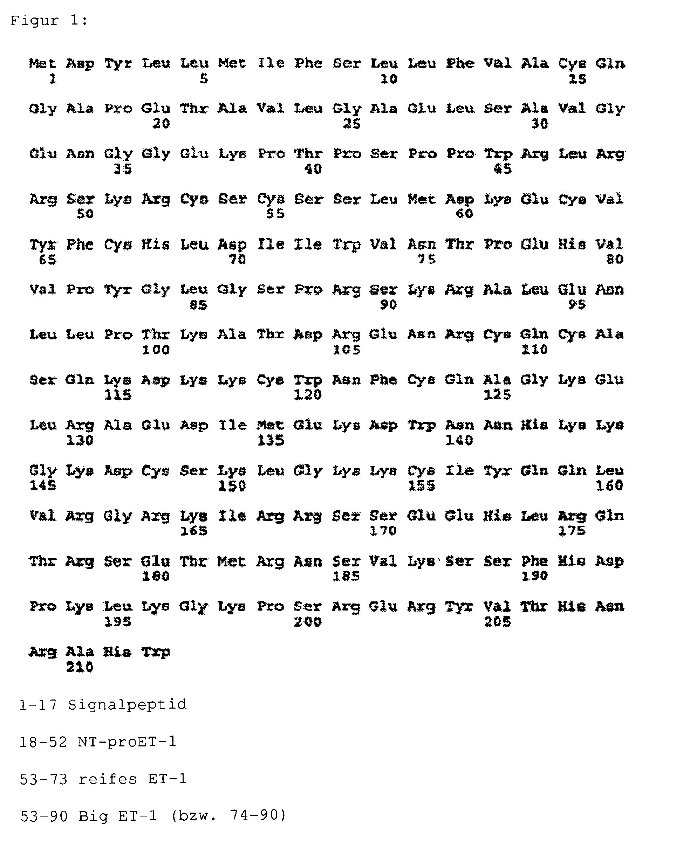

Die Erfindung betrifft ein Verfahren zur Diagnose und / oder Risikostratifizierung von Erkrankungen der Atemwege und Lunge, wobei eine Bestimmung des freien Fragment N-terminales proEndothelin (NT-proET-1; AS 18-52 des preproET gemäß Figur 1) oder Fragmenten davon, erfolgt.

Zwecks einer geeigneten Therapie, bedarf es einer frühen Diagnose und Differenzierung von Erkrankungen der Atemwege und Lunge bereits in der Notaufnahme in Verbindung mit der Notwendigkeit klinische Entscheidungen zu treffen.

Im Stand der Technik ist das reife ET-1 (21 AS) zur Diagnostik samt Vorläuferprotein Big-ET 1 (38AS, siehe Figur 1) beschrieben (Rossi GP, Seccia TM, Albertin G, Pessina AC. Measurement of endothelin: clinical and research use. Ann Clin Biochem 2000;37(Pt 5):608-26.2; Aubin P, Le Brun G, Moldovan F, Villette JM, Creminon C, Dumas J, et al. Sandwich-type enzyme immunoassay for big endothelin-I in plasma: concentrations in healthy human subjects unaffected by sex or posture. Clin Chem 1997;43: 64-70). Ferner ist bekannt, dass Endothelin-Prohormone zur Diagnose von Sepsis in WO 00/22439 bzw. EP 1121600 B1 der Anmelderin offenbart sind.

Ferner sind die C-terminalen ProEndothelin Fragmente (CTproET-1) mit den Aminosäuresequenzen 93-212 oder 168-212 des preproEndothelin (Figur 1, SEQ ID No. 1) zur Diagnose von Herz-Kreislauferkrankungen zur indirekten Bestimmung des Endothelin-1 oder des big - Endothelin-1 Gehaltes in EP 1564558 B1 beschrieben.

Nachteilig an den bekannten Diagnoseverfahren unter Verwendung der bisher bekannten Marker ist jedoch weiterhin, dass eine frühzeitige und vollständige Erfassung von Risikopatienten nicht ausreichend gelingt und daher eine Risikostratifizierung nicht ausreichend erfolgt. Eine der Erfindung zugrunde liegende Aufgabe besteht daher darin, ein Verfahren zur Risikostratifizierung von Herzerkrankungen sowie Erkrankungen der Atemwege und Lunge zu entwickeln, das eine verbesserte Erfassung von Risikopatienten ermöglicht.

Ferner ist nachteilig, dass im Stand der Technik zumeist keine hinreichende Sensitivität und/oder Spezifität der Marker erreicht wird.

Struck et al. (2005), Peptides, Vol. 26, Seiten 2482-2486 enthüllen die Messung des Vorläuferpeptids von Endothelin-1 in Plasma stellen aber keinen Zusammenhang des Peptids als Marker mit der Diagnose oder Stratifizierung von Lungenkrankheiten oder Erkrankungen der Atemwege her.

Eine weitere Aufgabe besteht daher darin, ein Verfahren zur Risikostratifizierung von Erkrankungen der Atemwege und Lunge bereitzustellen, wobei mindestens ein Marker oder eine Kombination von Markern eine hinreichende Sensitivität und Spezifität in einer in-vitro Diagnose aufweist.

Daher ist es Aufgabe der vorliegenden Erfindung ein Verfahren zur Diagnose und / oder Risikostratifizierung von Erkrankungen der Atemwege und Lunge bereitzustellen.

Die Aufgabe wird durch ein Verfahren zur Diagnose und Risikostratifizierung von Erkrankungen der Atemwege und Lunge gelöst, wobei eine Bestimmung des N-terminales proEndothelin (kurz: "NT-proET-1") oder Fragmenten und Teilpeptiden davon, erfolgt (nachstehend erfindungsgemäßes Verfahren).

Überraschender Weise weisen NT-proET-1 oder Fragmente und Teilpeptide davon, eine hohe Sensitivität und Spezifität für die Diagnose von Erkrankungen der Atemwege und Lunge auf (Siehe Beispiele und Figuren).

Im Rahmen dieser Erfindung wird unter "Erkrankungen der Atemwege und der Lunge" insbesondere "Infektionen der Lunge und Atemwege" und zwar solche Infektionen verstanden, die durch Bakterien, Viren, Pilze oder Parasiten verursacht werden, z.B. solche Indikationen wie tiefe Atemwegsinfektion (LRTI: Lower respiratory tract infections), Bronchitis, Pneumonie, Sarkoidose, Bronchiektasen, Nicht-kardiales Lungenödem.

Erfindungsgemäß bevorzugt sind zudem tiefe Atemwegsinfektion (LRTI: Lower respiratory tract infections), Bronchitis, putride Bronchitis, Pneumonie. Ganz besonders bevorzugt ist Pneumonie, insbesondere die ambulant erworbene Pneumonie (CAP: community associated pneumonie), tiefe Atemwegsinfektion (LRTI: Lower respiratory tract infections).

Im Rahmen dieser Erfindung wird unter Pneumonie eine akute oder chronische Erkrankung des Lungengewebes verstanden und dessen Infektion ist verursacht durch Bakterien, Viren oder Pilze, Parasiten, selten auch toxisch durch Inhalation giftiger Stoffe oder immunologisch. Für den Kliniker ist die Pneumonie eine Konstellation verschiedener Symptome (Fieber oder Hypothermie, Schüttelfrost, Husten, pleuritischer Thoraxschmerz, gesteigerte Sputumproduktion, erhöhte Atemfrequenz, Klopfschalldämpfung, Bronchialatmen, ohrnahe Rasselgeräusche, Pleurareiben) in Kombination mit mindestens einem auf dem Thorax-Röntgenbild erkennbaren Infiltrat (Harrisons Innere Medizin, herausgegeben von Manfred Dietel, Norbert Suttorp und Martin Zeitz, ABW Wissenschaftsverlag 2005).

Im Rahmen dieser Erfindung wird unter "Erkrankungen der Atemwege und der Lunge" insbesondere "chronische Erkrankungen der Lunge und Atemwege" verstanden und zwar solche Indikationen, wie Interstitielle Lungenerkrankungen und Lungenfibrosen, Chronisch obstruktive Lungenerkrankungen (COPD), insbesondere COPD Infekt-Exazerbationen, Asthma bronchiale, insbesondere Infekt-Exazerbationen bei Asthma bronchiale, Bronchialkarzinom. Ganz besonders bevorzugt ist COPD, insbesondere COPD Infekt-Exazerbationen.

COPD bezeichnet erfindungsgemäß eine Gruppe von chronischen Krankheiten, die durch Husten, vermehrten Auswurf und Atemnot bei Belastung gekennzeichnet sind. In erster Linie sind die chronisch-obstruktive Bronchitis und das Lungenemphysem zu nennen. Beide Krankheitsbilder sind dadurch gekennzeichnet, dass vor allem die Ausatmung (Exspiration) behindert ist. Eine umgangssprachliche Bezeichnung für das Hauptsymptom der COPD ist zudem "Raucherhusten". Die Erfindung ist besonders vorteilhaft bei akuten Exazerbationen.

Alle genannten Indikationen werden zudem z.B. im Pschyrembel, De Gruyter, 9. Auflage, Berlin 2004 beschrieben.

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Erkrankungen der Atemwege und Lunge, insbesondere von Infektionen und chronischen Entzündungen mit dem Ziel einen möglichst günstigen Verlauf der Krankheit zu ermöglichen. Eine erfindungsgemäße Risikostratifizierung erlaubt in Folge ein effektives Behandlungsverfahren zur Behandlung oder Therapie von Infektionen oder chronischen Erkrankungen der Atemwege und Lunge mittels Antibiotika.

Daher betrifft die Erfindung ebenfalls die Identifizierung von Patienten mit erhöhtem Risiko oder/und einer ungünstigen Prognose von Erkrankungen der Atemwege und Lunge und zwar bei symptomatischen und / oder asymptomatischen Patienten, insbesondere Notfallpatienten.

Besonders vorteilhaft kann insbesondere in Fällen der Notfall- und /oder Intensivmedizin mittels des erfindungsgemäßen Verfahren eine sichere Stratifizierung erfolgen. Das erfindungsgemäße Verfahren ermöglicht daher klinische Entscheidungen, die zu einem schnellen Therapieerfolg und zur Vermeidung von Todesfällen führen. Solche klinischen Entscheidungen umfassen ebenfalls weiterführende Behandlung mittels Arzneimitteln zur Behandlung oder Therapie von Erkrankungen der Atemwege und Lunge.

Daher betrifft die Erfindung ebenfalls ein Verfahren zur Diagnose und / oder Risikostratifizierung von Patienten von Erkrankungen der Atemwege und Lunge zur Durchführung von klinischen Entscheidungen, wie weiterführende Behandlung und Therapie mittels Arzneimitteln, vorzugsweise in der zeitlich kritischen Intensivmedizin oder Notfallmedizin, einschließlich der Entscheidung zur Hospitalisierung des Patienten.

In einer weiteren bevorzugten Ausführungsform betrifft das erfindungsgemäße Verfahren daher die Therapiesteuerung von Erkrankungen der Atemwege.

In einer weiteren bevorzugten Ausführungsform des erfindungsgemäßen Verfahrens erfolgt die zur Diagnose und / oder Risikostratifizierung zur Prognose, zur differentialdiagnostischen Früherkennung und Erkennung, zur Beurteilung des Schweregrades und zur therapiebegleitenden Verlaufsbeurteilung.

In einer weiteren bevorzugten Ausführungsform betrifft die Erfindung ein Verfahren zur in-vitro Diagnostik zur Früh- oder Differentialdiagnose oder Prognose von Erkrankungen der Atemwege und Lunge, wobei eine Bestimmung des Marker NT-proET-1 oder Fragmente und Teilpeptide davon, an einem zu untersuchenden Patienten durchgeführt wird.

In einer Ausführungsform des erfindungsgemäßen Verfahrens wird dem zu untersuchenden Patienten Körperflüssigkeit, vorzugsweise Blut entnommen, wahlweise Vollblut oder Serum oder erhältliches Plasma und die Diagnose erfolgt *in vitro*/*ex* vivo, d.h. außerhalb des menschlichen oder tierischen Körpers. Aufgrund der Bestimmung des Markers NT-proET-1 oder Fragmenten und Teilpeptiden davon, wird eine hohe Sensitivität und Spezifität erzielt (siehe Beispiele und Figuren) und anhand der vorhandenen Menge in mindestens einer Patientenprobe kann die Diagnose oder Risikostratifizierung erfolgen.

Im Rahmen dieser Erfindung wird unter "NT-proET-1" ein humanes Protein oder Polypeptid verstanden, das aus dem Preproendothelin erhalten werden kann und im Rahmen des Preproendothelins (SEQ ID No.1 und Figur 1) die freien Fragmente mit den Aminosäuren 18-52 umfassen und die Fragmente APETAVLGAELSAV (SEQ ID No. 2) Pos. 18-31 von preproET-1 (SEQ ID No.1 und Figur 1) und GENGGEKPTPSPPWRLRRSKR (SEQ ID No. 3) Pos. 32-52 von preproET-1 (SEQ ID No.1 und Figur 1). Ferner können diese erfindungsgemäßen Polypeptide posttranslationale Modifikationen aufweisen, wie Glykolisierung, Lip(o)idisierung oder Derivatisierungen.

In einer weiteren Ausführungsform kann die Bestimmung von NT-proET-1 oder Fragmenten und Teilpeptiden davon, zusätzlich mit weiteren Markern erfolgen und zwar vorzugsweise solche, die bereits auf Erkrankungen der Atemwege und Lunge hinweisen.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker, neurohormonaler Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker, insbesondere ein Nekrose von Herzmuskelgewebe anzeigender Marker und den Blutdruck beeinflussender Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin-2, pro-Endothelin-3, pro-Leptin, pro-Neuropeptid-Y, pro-Somatostatin, pro-Neuropeptid-YY, pro-Opiomelanocortin, pro-Adrenomedullin (proADM), pro-Vasopressin (proAVP) oder jeweils eine Teilsequenz davon.
Der ischämische Marker kann aus mindestens einem Marker aus der Gruppe Troponin I und T, CK-MB ausgewählt sein. Darüber hinaus kann der neurohormonale Marker mindestens ein weiteres natriuretisches Protein sein, insbesondere ANP (bzw. ANF), proANP, NT-proANP, BNP, proBNP oder jeweils eine Teilsequenz davon.

In einer weiteren Ausführungsform der Erfindung kann das erfindungsgemäße Verfahren im Rahmen einer in-vitro Diagnose mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen in einer diagnostischen Vorrichtung anhand eines Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test oder Point-of Care), sei es in Einzel- oder Multiparameterbestimmung. In einer besonders bevorzugten Ausführungsform handelt es sich um einen Selbsttest oder um eine Vorrichtung, die in der Notfalldiagnostik geeignet ist.

Ferner betrifft die Erfindung die Verwendung von NT-proET-1 oder Fragmente davon, zur Risikostratifizierung von Erkrankungen der Atemwege und Lunge und/oder zur in-vitro Diagnostik zur Früh- oder Differentialdiagnose oder Prognose von Erkrankungen der Atemwege und Lunge.

Eine weitere Aufgabe ist die Verwendung einer entsprechenden diagnostischen Vorrichtung zur Durchführung des erfindungsgemäßen Verfahrens.

Im Rahmen dieser Erfindung wird unter einer solchen diagnostischen Vorrichtung, insbesondere ein Array oder Assay verstanden (z.B. Immunoassay, ELISA etc.), im weitesten Sinne eine Vorrichtung zur Durchführung des erfindungsgemäßen Verfahrens.

Die Erfindung betrifft zudem die Verwendung eines Kits zur Diagnose und / oder Risikostratifizierung von Erkrankungen der Atemwege und Lunge, enthaltend Nachweisreagenzien zur Bestimmung von NT-proET-1 oder Fragmenten davon, ggfs. oben genannte weitere Marker. Solche Nachweisreagenzien umfassen z.B. Antikörper etc.

Nachfolgende Beispiele und Figuren dienen zur näheren Erläuterung der Erfindung, jedoch ohne die Erfindung auf diese Beispiele und Figuren zu beschränken.

### Beispiele und Figuren:

### Immunoassay

Es wurde ein Sandwichimmunoassay zur Messung von NT-proET-1 wie beschrieben in Struck et al (Struck J, Morgenthaler NG, Bergmann A. Proteolytic processing pattern of the endothelin-1 precursor in vivo. Peptides 2005; 26:2482-6) verwendet.

### Peptidsynthesen:

Abgeleitet von der bekannten Aminosäuresequenz von preproET-1 (Figur 1) wurden zwei Bereiche ausgewählt (Pos. 18-31, 32-52). Die Bereiche wurden, in einem Fall ergänzt um einen N-terminalen Cystein-Rest, nach Standardverfahren als lösliche Peptide chemisch synthetisiert, gereinigt, mittels Massenspektrometrie und Reversed Phase HPLC qualitätskontrolliert und in Aliquots lyophilisiert (Firma JPT, Berlin, Deutschland). Die Aminosäuresequenzen der Peptide lauten:
PAV15 CAPETAVLGAELSAV Pos. 18-31 von preproET-1 (Figur 1),
MPGC22 GENGGEKPTPSPPWRLRRSKRC Pos. 32-52 von preproET-1 (Figur 1).
Des Weiteren wurde ein Peptid synthetisiert, das den Bereich Pos. 18-52 von preproET-1 umfasst (PAR35 APETAVLGAELSAVGENGGEKPTPSPPWRLRRSKR).

### Konjugation und Immunisierung

Mittels MBS (m-Maleimidobenzoyl-N-hydroxysuccinimid Ester) wurden die Peptide PAV15 und MPGC22 an das Trägerprotein KLH (Keyhole limpet hemocyanin) konjugiert (s. Arbeitsanleitung "NHS-Esters-Maleimide Crosslinkers" Firma PIERCE, Rockford, IL, USA). Mit diesen Konjugaten wurden Schafe nach folgendem Schema immunisiert: Jedes Schaf erhielt initial 100 µg Konjugat (Massenangabe bezogen auf den Peptid-Anteil des Konjugats) und anschließend 4-wöchentlich je 50 µg Konjugat (Massenangabe bezogen auf den Peptid-Anteil des Konjugats). Beginnend mit dem vierten Monat nach Beginn der Immunisierung wurden 4-wöchentlich je Schaf 700 ml Blut abgenommen und daraus durch Zentrifugation Antiserum gewonnen. Konjugationen, Immunisierungen und Gewinnung von Antiseren wurden von der Firma MicroPharm, Carmarthenshire, UK, durchgeführt.

### Reinigung der Antikörper

In einem 1-Schritt Verfahren wurden aus den Antiseren, die beginnend mit dem vierten Monat nach der Immuniserung gewonnen worden waren, die Peptid-spezifischen Antikörper präpariert. Dazu wurden zunächst die Peptide PAV15 und MPGC22 an SulfoLink Gel gekoppelt (s. Arbeitsanleitung "SulfoLink Kit" Firma PIERCE, Rockford, IL, USA). Dabei wurden zur Kopplung je 5 mg Peptid pro 5 ml Gel angeboten.

Die Affinitätsreinigung von Peptid-spezifischen Antikörpern aus Schaf Antiseren gegen die Peptide wurde wie folgt durchgeführt:
Die Peptid-Säulen wurden zunächst dreimal im Wechsel mit je 10 ml Elutionspuffer (50 mM Citronensäure, pH 2.2) und Bindungspuffer (100 mM Natriumphosphat, 0.1% Tween, pH 6.8) gewaschen. 100 ml der Antiseren wurden über 0,2 µm filtriert und mit dem vorhandenen Säulenmaterial versetzt. Dazu wurde das Gel quantitativ mit 10 ml Bindungspuffer aus der Säule gespült. Die Inkubation erfolgte über Nacht bei Raumtemperatur unter Schwenken. Die Ansätze wurden quantitativ in Leersäulen (NAP 25, Pharmacia, entleert) überführt. Die Durchläufe wurden verworfen. Anschließend wurde mit 250 ml Bindungspuffer proteinfrei (Proteingehalt des Wascheluats < 0,02 A280 nm) gewaschen. Auf die gewaschene Säulen wurde Elutionspuffer gegeben, und es wurden Fraktionen ä 1 ml gesammelt. Von jeder Fraktion wurde der Proteingehalt mittels der BCA-Methode (s. Arbeitsanleitung Firma PIERCE, Rockford, IL, USA) bestimmt. Fraktionen mit Proteinkonzentrationen > 0.8 mg/ml wurden gepoolt. Nach Proteinbestimmung der Pools mittels der BCA-Methode ergaben sich Ausbeuten von 27 mg für den anti-PAV15 Antikörper und 35 mg für den anti- MPGC22 Antikörper.

### Markierung

Über NAP-5 Gelfiltrationssäulen (Pharmacia) wurden 500 µl des gereinigten anti-MPGC22 Antikörpers s.o.) in 1 ml 100 mM Kalium-Phosphatpuffer (pH 8,0) nach Arbeitsanleitung umgepuffert. Die Proteinkonzentationen der Antikörperlösung wurden mit 100 mM Kalium-Phosphatpuffer (pH 8,0) auf 1,5 mg/ml eingestellt.

Zur Chemilumineszenzmarkierung wurde der Antikörper wie folgt weiterbehandelt: 67 µl der Antikörperlösung wurden mit 10 µl MA70-Akridinium-NHS-Ester (1 mg/ml; Firma HOECHST Behring) versetzt und 15 Minuten bei Raumtemperatur inkubiert. Dann wurden 423 µl 1 M Glycin zugesetzt und weitere 10 Minuten inkubiert. Anschließend wurde der Markierungsanastz über eine NAP-5 Gelfiltrationssäule (Pharmacia) in 1 ml Laufmittel A (50 mM Kaliumphosphat, 100 mM NaCl, pH 7.4) nach Arbeitsanleitung umgepuffert und dabei von niedermolekularen Bestandteilen befreit. Zur Abtrennung letzter Reste nicht an Antikörper gebundenen Labels wurde eine Gelfiltrations-HPLC durchgeführt (Säule: Waters Protein Pak SW300). Die Probe wurde aufgetragen und bei einer Flußrate von 1 ml/min mit Laufmittel A chromatographiert. Mit einem Duchflußphotometer wurden die Wellenlängen 280 nm und 368 nm gemessen. Das Absorbtionsverhältnis 368 nm/280 nm als Maß für den Markierungsgrad des Antikörpers betrug am Peak 0.10 +/- 0.01. Die monomeren Antikörper enthaltenden Fraktionen (Retentionszeit 8-10 min) wurden gesammelt und in 3 ml 100 mM Natriumphosphat, 150 mM NaCl, 5% Bovines Serum Albumin, 0.1% Natrium Azid, pH 7.4, gesammelt.

### Kopplung

Bestrahlte 5 ml Polystyrolröhrchen (Firma Greiner) wurden mit gereinigtem anti-PAV15 Antikörper wie folgt beschichtet: Der Antikörper wurde in 50 mM Tris, 100 mM NaCl, pH 7.8 zu einer Konzentration von 6.6 µg/ml verdünnt. In jedes Röhrchen wurden 300 µl dieser Lösung pipettiert. Die Röhrchen wurden 20 Stunden bei 22°C inkubiert. Die Lösung wurde abgesaugt. Dann wurde jedes Röhrchen mit 4.2 ml 10 mM Natriumphosphat, 2% Karion FP, 0.3% Bovines Serum Albumin, pH 6.5 befüllt. Nach 20 Stunden wurde die Lösung abgesaugt. Schließlich wurden die Röhrchen in einem Vakuumtrockner getrocknet.

### Durchführung und Auswertung des Immunoassays

Als Standardmaterial diente Peptid PAR35, das seriell in Pferdenormalserum (Firma SIGMA) verdünnt wurde. Den so hergestellten Standards wurden Konzentrationen gemäß der Peptideinwaage zugeschrieben.
Der Sandwichimmunoassay wurde wie folgt angesetzt: In die jeweiligen mit Antikörpern beschichteten Teströhrchen wurden 50 µl Standards bzw. Proben sowie 200 µl Assaypuffer (100 mM Natriumphosphat, 150 mM NaCl, 5% Bovines Serum Albumin, 0.1% unspez. Schaf IgG, 0.1% Natrium Azid, pH 7.4), enthaltend 1 Million RLU (relative light units) des MA70-markierten Antikörpers, pipettiert. Es wurde 2 Stunden bei 22°C unter Schütteln inkubiert. Dann wurde 4 x mit je 1 ml Waschlösung (0.1% Tween 20) pro Röhrchen gewaschen, abtropfen gelassen und die am Röhrchen gebundene Chemilumineszenz in einem Luminometer (Firma BERTHOLD, LB952T; Basisreagenzien BRAHMS AG) vermessen. Unter Verwendung der Software MultiCalc (Spline Fit) wurden die NT-proET-1 Konzentrationen der Proben an der Standardkurve abgelesen.
Analyt, der mit dem beschriebenen Assay gemessen werden kann, wird bezeichnet als N-terminales proEndothelin-1 (NT-proET-1).

### Klinische Wertigkeit (Beispiele, die Patienten mit Herzerkrankungen betreffen, dienen als Referenz).

### Normalbereich

NT-proET-1 Konzentrationen wurden in Proben von gesunden Kontrollpersonen (n=200) bestimmt. Der Median lag bei 30,5 pmol/L, der kleinste gemessene Wert bei 2,0 der größte bei 53 pmol/L, die 95% Percentilen bei 16,3 bzw. 47,2 pmol/L.

### Herzinsuffizienz / Schweregrad

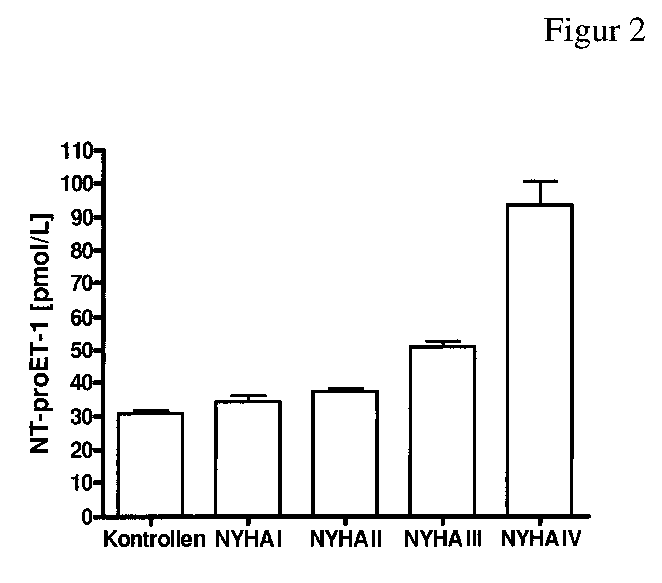

Es wurden NT-proET-1 Konzentrationen bei Patienten mit chronischer oder akut dekompensierter Herzinsuffizienz vermessen. NT-proET-1 Konzentrationen waren mit dem Schweregrad der Herzinsuffizienz assoziiert, insbesondere mit erhöhtem Schweregrad: Die Mediane der NT-proET-1 Konzentrationen bei den vier Schweregrad-Kategorien NYHA I-IV waren: 35,4, 37,3, 45,5 bzw. 83,9 pmol/L (s. Fig. 2).

### Chronische Herzinsuffizienz / Diagnose

Von einem Kollektiv von 316 Patienten mit chronischer Herzinsuffizienz sowie 200 gesunden Kontrollen wurden NT-proET-1 Werte bestimmt. Die Receiver-Operator-Characteristics Analyse ergab eine AUC von 0,72. Bei einem Cut-off-Wert von 49,7 pmol/L ergab sich eine Sensitivität von 34,3% bei einer Spezifität von 98%. Bei einem Cut-off-Wert von 47,2 pmol/L ergab sich eine Sensitivität von 38,5% bei einer Spezifität von 95%.

### Chronische Herzinsuffizienz / Prognose

Von einem Kollektiv von 316 Patienten mit chronischer Herzinsuffizienz wurden NT-proET-1 Werte bestimmt. Die Patienten wurden über einen mittleren Zeitraum von 360 Tagen beobachtet. In diesem Zeitraum starben 42 Patienten, 274 überlebten. Durch Receiver-Operator-Characteristics Analyse wurde der beste Cut-off-Wert (definiert als das größte Produkt aus Sensitivität und Spezifität) zur Prognose der Mortalität ermittelt: 51,3 pmol/L. Bei diesem Cut-off-Wert betrug die Sensitivität der Prognose 71,4%, die Spezifität betrug 74,5%. Die Likelihood Ratio zu versterben betrug bei einem Cut-off-Wert von 51,3 pmol/L: 2,8.

| | <51,3 pmol/L | >51,3 pmol/L |
|---|---|---|
| Überlebende | 204 | 70 |
| Tote | 12 | 30 |

### Akute Herzinsuffizienz / Diagnose

Von einem Kollektiv von 125 Patienten mit akuter Atemnot wurden NT-proET-1 Werte bestimmt. Von den 125 Patienten hatten 69 Patienten Herzinsuffizienz. Die Receiver-Operator-Characteristics Analyse für die Differentialdiagnose der Herzinsuffizienz ergab eine AUC von 0,72. Bei einem Cut-off-Wert von 111 pmol/L ergab sich eine Sensitivität von 10,2% bei einer Spezifität von 98%. Bei einem Cut-off-Wert von 90,5 pmol/L ergab sich eine Sensitivität von 17,5% bei einer Spezifität von 95%.

### Akute Herzinsuffizienz / Prognose

Von einem Kollektiv von 69 Patienten mit akut dekompensierter Herzinsuffizienz wurden NT-proET-1 Werte bestimmt. Die Patienten wurden über einen Zeitraum von 360 Tagen beobachtet. In diesem Zeitraum starben 21 Patienten, 48 überlebten. Durch Receiver-Operator-Characteristics Analyse wurde der beste Cut-off-Wert (definiert als das größte Produkt aus Sensitivität und Spezifität) zur Prognose der Mortalität ermittelt: 64 pmol/L. Bei diesem Cut-off-Wert betrug die Sensitivität der Prognose 66,6%, die Spezifität betrug 68,7%. Die Likelihood Ratio zu versterben betrug bei einem Cut-off-Wert von 64 pmol/L: 2,0.

| | <64 pmol/L | >64 pmol/L |
|---|---|---|
| Überlebende | 33 | 15 |
| Tote | 7 | 14 |

### Myokardinfarkt / Prognose

Von 246 Patienten mit akutem Herzinfarkt wurden drei Tage nach Eintreten des Herzinfarkts Proben gewonnen und es wurde NT-proET-1 gemessen. Die Patienten wurden über einen Zeitraum von 60 Tagen beobachtet. In diesem Zeitraum hatten 220 Patienten kein adverses Ereignis, 26 starben oder wurden wegen Herzinsuffizienz rehospitalisiert. Durch Receiver-Operator-Characteristics Analyse (AUC = 0,70) wurde der beste Cut-off-Wert (definiert als das größte Produkt aus Sensitivität und Spezifität) zur Prognose der Mortalität bzw. Rehospitalisierung wegen Herzinsuffizienz ermittelt: 63,9 pmol/L. Bei diesem Cut-off-Wert betrug die Sensitivität der Prognose 65,4%, die Spezifität betrug 73,6%. Die Likelihood Ratio eines adversen Ereignisses betrug bei einem Cut-off-Wert von 63,9 pmol/L: 2.5.

| | <63,9 pmol/L | >63,9 pmol/L |
|---|---|---|
| keine adverses Ereignis | 162 | 58 |
| Tot/Herzinsuffizienz | 9 | 17 |

### Pneumonie / Schweregrad und Prognose

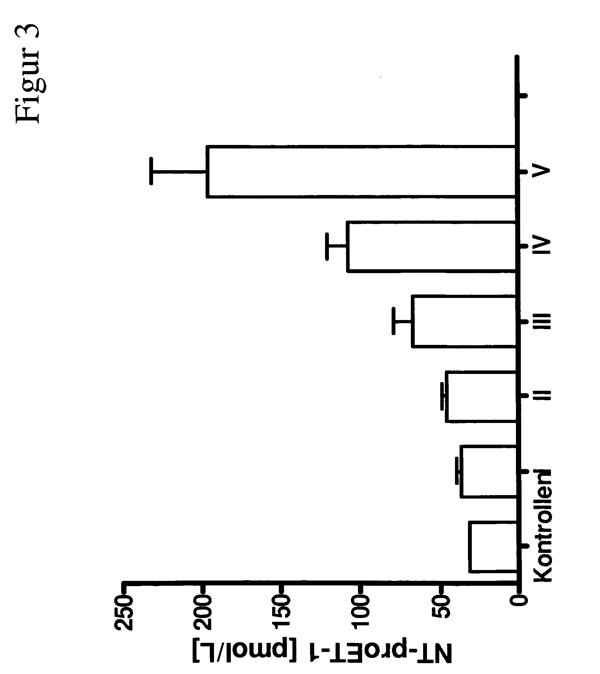

Von 142 Patienten mit ambulant erworbener Pneumonie wurden bei Aufnahme ins Krankenhaus Proben gewonnen und es wurde NT-proET-1 gemessen. Die Patienten wurden über einen Zeitraum von 70 Tagen beobachtet. In diesem Zeitraum starben 10 Patienten. NT-proET-1 Konzentrationen stiegen mit dem PSI (Pneumonia Severity Index), einem Score für den Schweregrad der Erkrankung (Figur 3) und waren im Median mit 53 pmol/L gegenüber gesunden Kontrollen (30,5 pmol/L) erhöht. Für die Prognose der Mortalität erbrachte die Receiver-Operator-Characteristics Analyse eine AUC von 0,89. Durch Receiver-Operator-Characteristics Analyse wurde der beste Cut-off-Wert (definiert als das größte Produkt aus Sensitivität und Spezifität) zur Prognose der Mortalität ermittelt: 77 pmol/L. Bei diesem Cut-off-Wert betrug die Sensitivität der Prognose 100%, die Spezifität betrug 81,2%. Die Likelihood Ratio der Mortalität betrug bei einem Cut-off-Wert von 77 pmol/L: 5.5.

| | <77 pmol/L | >77 pmol/L |
|---|---|---|
| keine adverses Ereignis | 108 | 24 |
| Tot | 0 | 10 |

### Exacerbierte COPD

Von 53 Patienten mit chronisch obstruktiver Lungenerkrankung und gleichzeitiger Infektion der unteren Atemwege wurde NT-proET-1 gemessen. Mit im Median 47 pmol/L waren diese Patienten gegenüber gesunden Kontrollen (30,5 pmol/L) erhöht, lagen jedoch niedriger als Pneumonie Patienten (s.o. 53 pmol/L).

Tabelle: Klassifikation der New York Heart Association (NYHA)
- NYHA I: Keine körperliche Einschränkung. Alltägliche körperliche Belastung verursacht keine inadäquate Erschöpfung, Rhythmusstörungen, Luftnot oder Angina Pectoris.
- NYHA II: Leichte Einschränkung der körperlichen Belastbarkeit. Keine Beschwerden in Ruhe. Erschöpfung, Rhythmusstörungen, Luftnot oder Angina pectoris bei alltäglicher körperlicher Belastung.
- NYHA: IIIHöhergradige Einschränkung der körperlichen Leistungsfähigkeit bei gewohnter Tätigkeit. Keine Beschwerden in Ruhe. Erschöpfung, Rhythmusstörungen, Luftnot oder Angina pectoris bei geringer körperlicher Belastung.
- NYHA IV: Beschwerden bei allen körperlichen Aktivitäten und in Ruhe. Bettlägrigkeit.

### Beschreibung der Figuren:

Figur 1 zeigt die Aminosäuresequenz (AS) von preproEndothelin-1 mit den Abschnitten 1-17 Signalpeptid, 18-52 NT-proET-1, 53-73 reifes ET-1, 53-90 Big ET-1 (bzw. 74-90).
Figur 2:
   NT-proET-1 Konzentrationen in Abhängigkeit vom Schweregrad der Herzinsuffizienz. Dargestellt sind die Mittelwerte (+SEM) von gesunden Kontrollen (n=200), sowie von Herzinsuffizienzpatienten gruppiert nach NYHA Schweregrad (NYHA I: n=22, NYHA II: n=126, NYHA III: n=132, NYHA 4: n=17).
Figur 3:
   NT-proET-1 Konzentrationen in Abhängigkeit vom Schweregrad der Pneumonie. Dargestellt sind die Mittelwerte (+SEM) von gesunden Kontrollen (n=200), sowie von Pneumoniepatienten gruppiert nach Pneumonia Severity Index (PSI I: n=36, PSI II: n=44, PSI III: n=25, PSI IV: n=19, PSI V: n=18).

## Patentansprüche

1. Verfahren zur in-vitro Diagnose und / oder Risikostratifizierung von Erkrankungen der Lunge und Atemwege, **dadurch gekennzeichnet, dass** eine Bestimmung des freien Fragments 18-52 aus SEQ ID No 1 (NT-proET-1) oder ein Fragment ausgewählt aus SEQ ID No. 2, SEQ ID No. 3 in einer Körperflüssigkeitsprobe von einem zu untersuchenden Patienten erfolgt.

2. Verfahren zur Diagnose und / oder Risikostratifizierung von Erkrankungen der Lunge und Atemwege nach Anspruch 1, **dadurch gekennzeichnet, dass** diese ausgewählt sind aus Infektionen verursacht durch Bakterien, Viren, Pilze oder Parasiten, insbesondere tiefe Atemwegsinfektion (LRTI: Lower respiratory tract infections), Bronchitis, Pneumonie, Sarkoidose, Bronchiektasen, Nicht-kardiales Lungenödem und / oder chronische Erkrankungen der Lunge und Atemwege, insbesondere Interstitielle Lungenerkrankungen und Lungenfibrosen, Chronisch obstruktive Lungenerkrankungen (COPD), insbesondere COPD Infekt-Exazerbationen, Asthma bronchiale, insbesondere Infekt-Exazerbationen bei Asthma bronchiale.

3. Verfahren zur Diagnose und / oder Risikostratifizierung nach einem der vorherigen Ansprüche, zur Identifizierung von Patienten mit erhöhtem Risiko oder/und einer ungünstigen Prognose von Erkrankungen der Lunge und Atemwege.

4. Verfahren zur Diagnose und / oder Risikostratifizierung nach einem der vorherigen Ansprüche, wobei der Patient ein symptomatischer und / oder asymptomatischer Patient, insbesondere ein Notfallpatient ist.

5. Verfahren zur Diagnose und / oder Risikostratifizierung nach einem der vorherigen Ansprüche, zur Therapiesteuerung von Erkrankungen der Lunge und Atemwege, insbesondere in der Intensivmedizin oder Notfallmedizin.

6. Verfahren zur Diagnose und / oder Risikostratifizierung von Erkrankungen der Lunge und Atemwege nach einem der vorherigen Ansprüche zur Durchführung von klinischen Entscheidungen, insbesondere weiterführende Behandlungen und Therapien mittels Arzneimitteln, insbesondere in der Intensivmedizin oder Notfallmedizin, einschließlich der Entscheidung zur Hospitalisierung des Patienten.

7. Verfahren zur Diagnose und / oder Risikostratifizierung von Erkrankungen der Lunge und Atemwege nach einem der vorherigen Ansprüche zur Prognose, zur differentialdiagnostischen Früherkennung und Erkennung, zur Beurteilung des Schweregrades und zur therapiebegleitenden Verlaufsbeurteilung.

8. Verfahren nach einem der vorherigen Ansprüche,
**dadurch gekennzeichnet,**
**dass** zusätzlich eine Bestimmung mindestens eines weiteren Markers ausgewählt aus der Gruppe inflammatorischer Marker, cardiovaskulärer Marker, neurohormonaler Marker oder ischämischer Marker durchgeführt wird.

9. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

10. Verfahren nach einem der vorherigen Ansprüche, **dadurch gekennzeichnet, dass** der cardiovaskuläre Marker aus mindestens einem Marker aus der Gruppe Kreatinkinase, Myoglobin, Myeloperoxidase, natriuretisches Protein, insbesondere ANP (bzw. ANF), proANP, NT-proANP, BNP, proBNP, NT-proBNP oder jeweils eine Teilsequenz davon, kardiale Troponin, CRP sowie Kreislaufregulierende (Pro)Hormone, wie pro-Gastrin-Releasing Peptid (proGRP), pro-Endothelin-2, pro-Endothelin-3, pro-Leptin, pro-Neuropeptid-Y, pro-Somatostatin, pro- Neuropeptid-YY, pro-Opiomelanocortin, pro-Adrenomedullin (proADM), pro-Vasopressin (proAVP) oder jeweils eine Teilsequenz davon ausgewählt ist.

11. Verfahren nach einem der vorherigen Ansprüche, **dadurch gekennzeichnet, dass** der ischämische Marker aus mindestens einem Marker aus der Gruppe Troponin I und T, CK-MB ausgewählt ist.

12. Verfahren nach einem der vorherigen Ansprüche, **dadurch gekennzeichnet, dass** der neurohormonale Marker mindestens ein natriuretisches Protein ist, insbesondere ANP (bzw. ANF), proANP, NT-proANP, BNP, proBNP, NT-proBNP oder eine Teilsequenz davon ist.

13. Verfahren nach einem der vorherigen Ansprüche,
**dadurch gekennzeichnet,**
**dass** parallele oder simultane Bestimmungen der Marker durchgeführt werden.

14. Verfahren nach einem der vorherigen Ansprüche,
**dadurch gekennzeichnet,**
**dass** die Bestimmungen an mindestens einer Patientenprobe durchgeführt werden.

15. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

16. Verwendung des freien Fragments 18-52 aus SEQ ID No 1 (NT-proET-1) oder ein Fragment ausgewählt aus SEQ ID No. 2, SEQ ID No. 3, zur Diagnose und / oder Risikostratifizierung von Erkrankungen der Lunge und Atemwege und ggfs. weitere Marker nach einem der Ansprüche 8 bis 12.

17. Verwendung eines Kits zur Diagnose und / oder Risikostratifizierung von Erkrankungen der Lunge und Atemwege enthaltend Nachweisreagenzien zur Bestimmung des freien Fragments 18-52 aus SEQ ID No 1 (NT-proET-1) oder ein Fragment ausgewählt aus SEQ ID No. 2, SEQ ID No. 3, und ggfs. weitere Marker nach einem der Ansprüche 8 bis 12 und Hilfsmittel.

## Claims

1. A method for the in-vitro diagnosis and/or risk stratification of diseases of the lungs and respiratory tract, **characterized in that** a determination of the free fragment 18-52 from SEQ ID No. 1 (NT-proET-1), or a fragment selected from SEQ ID No. 2, SEQ ID No. 3 in a sample of body fluid, is carried out from a patient to be examined.

2. The method for the diagnosis and/or risk stratification of diseases of the lungs and respiratory tract according to claim 1, **characterized in that** they are selected from infections cause by bacteria, viruses, fungi or parasites, particularly lower respiratory tract infections (LRTI), bronchitis, pneumonia, sarcoidosis, bronchiectases, noncardiac pulmonary edema and/or chronic diseases of the lungs and respiratory tract, particularly interstitial lung diseases and pulmonary fibrosis, chronic obstructive pulmonary disease (COPD), particularly COPD infection exacerbations, bronchial asthma, particularly infection exacerbations with bronchial asthma.

3. A method for the diagnosis and/or risk stratification according to any one of the preceding claims, for the identification of patients that are at increased risk and/or have an unfavorable prognosis for diseases of the lungs and respiratory tract.

4. A method for the diagnosis and/or risk stratification according to any one of the preceding claims, wherein the patient is a symptomatic and/or asymptomatic patient, particularly an emergency patient.

5. A method for the diagnosis and/or risk stratification according to any one of the preceding claims, for the control of the therapy of diseases of the lungs and respiratory tract, particularly in intensive care or emergency care.

6. A method for the diagnosis and/or risk stratification of diseases of the lungs and respiratory tract according to any one of the preceding claims, for the execution of clinical decisions, particularly advanced treatments and therapies using drugs, particularly in intensive care or emergency care, to include the decision to hospitalize the patient.

7. A method for the diagnosis and/or risk stratification of diseases of the lungs and respiratory tract according to any one of the preceding claims, for the prognosis, early detection and detection by differential diagnosis, assessment of the severity, and assessment of the course of the disease concomitant with the therapy.

8. A method according to any one of the preceding claims, **characterized in that** additionally a determination of at least one further marker is carried out, selected from the group of inflammatory markers, cardiovascular markers, neurohormonal markers, or ischemic markers.

9. A method according to any one of the preceding claims, **characterized in that** the inflammatory marker is selected from at least one marker of the group of C-reactive protein (CRP), cytokines such as TNF-alpha, interleukines such as IL-6, procalcitonin (1-116, 3-116), and adhesion molecules such as VCAM or ICAM.

10. A method according to any one of the preceding claims, **characterized in that** the cardiovascular marker is selected from at least one marker of the group creatine kinase, myoglobin, myeloperoxidase, natriuretic protein, particularly ANP (or ANF), proANP, NT-proANP, BNP, proBNP, NT-proBNP, or a partial sequence thereof, cardiac troponin, CRP, and also regulatory (pro)hormones, such as pro-gastrin releasing peptide (pro-GRP), pro-endothelin-2, pro-endothelin-3, pro-leptin, pro-neuropeptide Y, pro-somatostatin, pro-neuropeptide YY, pro-opionmelanocortin, pro-adrenomedullin (pro-ADM), pro-vasopressin (pro-AVP), or a partial sequence thereof.

11. A method according to any one of the preceding claims, **characterized in that** the ischemic marker is selected from at least one marker of the group troponin I and T, CK-MB.

12. A method according to any one of the preceding claims, **characterized in that** the neurohormonal marker is at least one natriuretic protein, particularly ANP (or ANF), proANP, NT-proANP, BNP, proBNP, NT-proBNP, or a partial sequence thereof.

13. A method according to any one of the preceding claims, **characterized in that** parallel or simultaneous determinations of the markers are carried out.

14. A method according to any one of the preceding claims, **characterized in that** the determinations are carried out using at least one patient sample

15. A method according to any one of the preceding claims, **characterized in that** the determinations are carried out using a rapid test, particularly in individual or multiple parameter determinations.

16. Use of the free fragment 18-52 from SEQ ID No. 1 (NT-proET-1), or a fragment selected from SEQ ID No. 2, SEQ ID No. 3, for the diagnosis and/or risk stratification of diseases of the lungs and respiratory tract, and optionally further markers, according to any one of claims 8 to 12.

17. Use of a kit for the diagnosis and/or risk stratification of diseases of the lungs and respiratory tract, comprising detection reagents for the determination of the free fragment 18-52 from SEQ ID No. 1 (NT-proET-1), or a fragment selected from SEQ ID No. 2, SEQ ID No. 3, and optionally further markers, according to any one of claims 8 to 12 and auxiliary agents.

## Revendications

1. Méthode pour le diagnostic et/ou la stratification du risque *in vitro* de maladies des poumons et du tractus respiratoire, **caractérisée par le fait qu'**une détermination du fragment libre 18-52 provenant de SEQ ID No. 1 (NT-proET-1), ou d'un fragment choisi parmi SEQ ID No. 2, SEQ ID No. 3 dans un échantillon de liquide organique, est mise en oeuvre à partir d'un patient à examiner.

2. Méthode pour le diagnostic et/ou la stratification du risque de maladies des poumons et du tractus respiratoire selon la revendication 1, **caractérisée par le fait qu'**elles sont choisies parmi des infections provoquées par des bactéries, des virus, des champignons ou des parasites, en particulier des infections du tractus respiratoire inférieur (LRTI), une bronchite, des pneumonies, une sarcoïdose, des bronchiectasies, un oedème pulmonaire non cardiaque et/ou des maladies chroniques des poumons et du tractus respiratoire, en particulier des maladies interstitielles du poumon et une fibrose pulmonaire, une maladie pulmonaire obstructive chronique (COPD), en particulier des exacerbations infectieuses d'une COPD, l'asthme, en particulier des exacerbations infectieuses avec de l'asthme.

3. Méthode pour le diagnostic et/ou la stratification du risque selon l'une quelconque des revendications précédentes, pour l'identification de patients qui présentent un risque accru et/ou ont un pronostic défavorable pour des maladies des poumons et du tractus respiratoire.

4. Méthode pour le diagnostic et/ou la stratification du risque selon l'une quelconque des revendications précédentes, le patient étant un patient symptomatique et/ou asymptomatique, en particulier un patient de services d'urgence.

5. Méthode pour le diagnostic et/ou la stratification du risque selon l'une quelconque des revendications précédentes, pour le contrôle de la thérapie de maladies des poumons et du tractus respiratoire, en particulier en soins intensifs ou en soins d'urgence.

6. Méthode pour le diagnostic et/ou la stratification du risque de maladies des poumons et du tractus respiratoire selon l'une quelconque des revendications précédentes, pour la prise de décisions cliniques, en particulier de traitements avancés et de thérapies à l'aide de médicaments, en particulier en soins intensifs ou en soins d'urgence, pour comprendre la décision d'hospitalisation du patient.

7. Méthode pour le diagnostic et/ou la stratification du risque de maladies des poumons et du tractus respiratoire selon l'une quelconque des revendications précédentes, pour le pronostic, la détection précoce et la détection par un diagnostic différentiel, une estimation de la sévérité et une estimation de l'évolution de la maladie, concomitants à la thérapie.

8. Méthode selon l'une quelconque des revendications précédentes, **caractérisée par le fait qu'**une détermination supplémentaire d'au moins un marqueur supplémentaire est mise en oeuvre, choisie parmi le groupe des marqueurs inflammatoires, des marqueurs cardiovasculaires, des marqueurs neurohormonaux ou des marqueurs ischémiques.

9. Méthode selon l'une quelconque des revendications précédentes, **caractérisée par le fait que** le marqueur inflammatoire est choisi parmi au moins un marqueur du groupe de la protéine C réactive (CRP), des cytokines telles que TNF-alpha, des interleukines telles que IL-6, de la procalcitonine (1-116, 3-116) et des molécules d'adhésion telles que VCAM ou ICAM.

10. Méthode selon l'une quelconque des revendications précédentes, **caractérisée par le fait que** le marqueur cardiovasculaire est choisi parmi au moins un marqueur du groupe créatine kinase, myoglobine, myéloperoxydase, protéine natriurétique, en particulier ANP (ou ANF), proANP, NT-proANP, BNP, proBNP, NT-proBNP, ou une séquence partielle de ceux-ci, la troponine cardiaque, CRP et également des (pro)hormones régulatrices, telles que le peptide de libération de la pro-gastrine (pro-GRP), la pro-endothéline-2, la pro-endothéline-3, la pro-leptine, le pro-neuropeptide Y, la pro-somatostatine, le pro-neuropeptide YY, la pro-opionmélanocortine, la pro-adrenomédulline (pro-ADM), la pro-vasopressine (pro-AVP), ou une séquence partielle de ceux-ci.

11. Méthode selon l'une quelconque des revendications précédentes, **caractérisée par le fait que** le marqueur ischémique est choisi parmi au moins un marqueur du groupe troponine I et T, CK-MB.

12. Méthode selon l'une quelconque des revendications précédentes, **caractérisée par le fait que** le marqueur neurohormonal est au moins une protéine natriurétique, en particulier ANP (ou ANF), proANP, NT-proANP, BNP, proBNP, NT-proBNP ou une séquence partielle de ceux-ci.

13. Méthode selon l'une quelconque des revendications précédentes, **caractérisée par le fait que** des déterminations parallèles ou simultanées des marqueurs sont mises en oeuvre.

14. Méthode selon l'une quelconque des revendications précédentes, **caractérisée par le fait que** les déterminations sont mises en oeuvre à l'aide d'au moins un échantillon de patient.

15. Méthode selon l'une quelconque des revendications précédentes, **caractérisée par le fait que** les déterminations sont mises en oeuvre à l'aide d'un test rapide, en particulier dans des déterminations à paramètre individuel ou multiple.

16. Utilisation du fragment libre 18-52 provenant de SEQ ID No. 1 (NT-proET-1), ou d'un fragment choisi parmi SEQ ID No. 2, SEQ ID No. 3, pour le diagnostic et/ou la stratification du risque de maladies des poumons et du tractus respiratoire, et facultativement, des marqueurs supplémentaires, selon l'une quelconque des revendications 8 à 12.

17. Utilisation d'une trousse pour le diagnostic et/ou la stratification du risque de maladies des poumons et du tractus respiratoire, comprenant des réactifs de détection pour la détermination du fragment libre 18-52 provenant de SEQ ID No. 1 (NT-proET-1), ou d'un fragment choisi parmi SEQ ID No. 2, SEQ ID No. 3, et facultativement des marqueurs supplémentaires, selon l'une quelconque des revendications 8 à 12, et des agents auxiliaires.
